(19) Europäisches Patentamt

**European Patent Office**

**Office européen des brevets**

(11) Numéro de publication: **0 315 533 B1**

(12) **FASCICULE DE BREVET EUROPEEN**

(45) Date de publication de fascicule du brevet: **02.09.92**   (51) Int. Cl.⁵: **C07C 233/20**, C07C 231/12

(21) Numéro de dépôt: **88402759.0**

(22) Date de dépôt: **03.11.88**

(54) **Acryloylamino-2 méthoxy-2 acétate de méthyle cristallisé pur et son procédé d'obtention.**

(30) Priorité: **04.11.87 FR 8715291**

(43) Date de publication de la demande:
**10.05.89 Bulletin 89/19**

(45) Mention de la délivrance du brevet:
**02.09.92 Bulletin 92/36**

(84) Etats contractants désignés:
**AT BE CH DE ES FR GB GR IT LI LU NL SE**

(56) Documents cités:
**EP-A- 0 138 025**
**US-A- 443 623**

(73) Titulaire: **SOCIETE FRANCAISE HOECHST Société anonyme dite:**
**3, avenue du Général de Gaulle**
**F-92800 Puteaux(FR)**

(72) Inventeur: **Christidis, Yani**
**53, Boulevard de la Villette**
**F-75019 Paris(FR)**
Inventeur: **Sidot, Christian**
**Résidence des Fleurs Avenue Foch**
**F-95460 Ezanville(FR)**

(74) Mandataire: **Rinuy, Santarelli**
**14, avenue de la Grande Armée**
**F-75017 Paris(FR)**

EP 0 315 533 B1

Rank Xerox (UK) Business Services

## Description

La présente invention concerne l'acryloylamino-2 méthoxy-2 acétate de méthyle cristallisé pur et son procédé d'obtention.

L'acryloylamino-2 méthoxy-2 acétate de méthyle, désigné par la suite MAGME, est un monomère connu plurifonctionnel couramment utilisé comme monomère réticulant.

Pour l'obtenir, on a proposé :

. D'éthérifier avec du méthanol, en milieu acide, l'acrylamidoglycolate de méthyle issu de la condensation de l'acrylamide avec l'hydroxy-2 méthoxy-2 acétate de méthyle (cf brevet canadien n° 1.209.583);

. D'éthérifier et d'estérifier simultanément, en milieu acide, l'acide acrylamidoglycolique cristallisé avec une molécule d'eau, avec du méthanol (cf brevet européen n° 0.020.000 et brevet des Etats-Unis d'Amérique n° 4.656.308).

Toutefois, ces procédés connus conduisent à du MAGME brut contenant de nombreuses impuretés et notamment de l'acrylamide extrêmement gênant lors de son emploi dans certaines applications.

Or, la demanderesse a découvert avec étonnement un nouveau procédé d'obtention du MAGME permettant d'accéder facilement et avec un bon rendement à un produit cristallisé pur.

Ce procédé est caractérisé en ce que l'on fait réagir, en milieu anhydre, de l'acide acrylamidoglycolique (désigné par la suite AAG) cristallisé pur anhydre avec du méthanol en présence de chlorure d'hydrogène, que l'on neutralise ensuite le milieu réactionnel avec un hydrogènocarbonate de métal alcalin puis que l'on filtre les sels minéraux et qu'enfin l'on isole le MAGME cristallisé pur par reprise du milieu réactionnel concentré sous vide avec du trichloro-1,1,1 éthane.

Dans des conditions préférentielles de mise en oeuvre de l'invention, le procédé ci-dessus décrit est réalisé de la manière suivante :

. On effectue toutes les opérations du procédé à une température inférieure à 35°C;

. On fait réagir 1 mole d'AAG cristallisé pur anhydre avec plus de 10 moles de méthanol en présence de 1 mole de chlorure d'hydrogène anhydre à une température comprise entre 30 et 35°C;

. On neutralise le milieu réactionnel avec de l'hydrogénocarbonate de sodium;

. En fin de réaction, on élimine le méthanol en excès et l'eau formée d'abord par distillation sous vide à une température inférieure à 35°C puis on achève cette élimination par distillation azéotropique sous vide avec du trichloro-1,1,1, éthane à une température inférieure à 35°C;

. On recristallise si nécessaire le MAGME dans du trichloro-1,1,1, éthane.

L'acide acrylamidoglycolique cristallisé pur anhydre utilisé comme produit de départ peut être obtenu par le procédé décrit dans la demande de brevet européen n° 248 481.

Le MAGME obtenu par le procédé de la présente invention se présente sous la forme d'un produit cristallisé, incolore, possédant un point de fusion net de 76°C et un indice de double liaison conforme à la théorie. En outre, l'analyse de ce produit, soit par microanalyse, soit par chromatographie liquide à hautes performances, soit par analyse thermique différentielle, soit enfin par RMN du proton à 200 MHz en solution dans le chloroforme deutéré ne révèle aucune impureté. De ce fait, ce produit présente une excellente stabilité et il est particulièrement apte à être utilisé dans ses applications connues.

Les exemples donnés ci-après illustrent l'invention sans toutefois la limiter.

EXEMPLE 1

On dissout à la température ambiante 290 g(2 moles) de AAG cristallisé pur anhydre obtenu entre autre selon l'exemple 1 de la demande de brevet européen rappelée ci-dessus et 3,2g de paraméthoxyphénol dans 897 g(28 moles) de méthanol anhydre.

Dans cette solution maintenue à 30-35°C, par un léger refroidissement extérieur, on introduit en une heure, sous agitation, 73g (2 moles) de chlorure d'hydrogène anhydre puis on abandonne la solution 2 heures sous agitation à 30°C avant de la neutraliser lentement avec 185g (2,2 moles) d'hydrogénocarbonate de sodium. Après 30 minutes d'agitation, on élimine les sels minéraux par filtration puis le filtrat est concentré sous un vide d'environ 13 mbar à une température inférieure à 30°C. On recueille ainsi 530g d'un mélange eau-méthanol. On achève ensuite l'élimination des solvants réactionnels en introduisant dans le milieu réactionnel 1350 g de trichloro-1,1,1 éthane puis en soumettant la solution obtenue à une distillation azéotropique sous vide (13 mbar) à une température inférieure à 35°C en introduisant en continu du trichloro-1,1,1 éthane neuf à un débit sensiblement identique à celui du distillat. Après l'introduction de 1350g de trichloro-1,1,1 éthane on arrête la distillation et on refroidit le milieu réactionnel. Le produit attendu cristallise spontanément. On l'essore et on le sèche à poids constant sous vide à 30°C.

On obtient ainsi 267g (1,54 mole) de MAGME cristallisé, incolore, présentant un point de fusion de 76°C, soit un rendement de 77% de la théorie calculée par rapport à l'AAG mis en oeuvre.

| Microanalyse | | C% | H% | N% | O% |
|---|---|---|---|---|---|
| $C_7H_{11}NO_4$ PM = 173,2 | calculés trouvés | 48,55 48,6 | 6,4 6,5 | 8,09 8,1 | 36,96 |

Dosage de double liaison : 5,72 ± 0,04 méq/g, soit 99±1% de la théorie.

Dosage d'eau : inférieur à 0,1%

Chromatographie liquide à hautes performances : 1 seul pic représentant 99,5% de MAGME.

Analyse thermique différentielle : PF = 76°C

Analyse physique infrarouge : Spectre conforme à la structure

Analyse physique RMN du proton à 200 MHz en solution dans le chloroforme deutéré :

. 6,82 ppm, d, 1H, J = 9Hz, NH,

6,65 ppm, d, 1H, J = 9Hz, CH-N,

6,40 ppm,dd, 1H, J = 17Hz et J = 1,5 Hz, $H_2C=$,

6,20 ppm,dd, 1H, J = 17HZ et J = 10 Hz,CH-CO,

5,78 ppm,dd,1H, J = 10Hz et J = 1,5 Hz, $H_2C=$,

3,83 ppm, s, 3H,COOCH$_3$,

3,48 ppm, s 3H,OCH$_3$.

EXEMPLE 2

On reproduit l'exemple 1 en remplaçant l'acide acrylamidoglycolique cristallisé pur anhydre par une quantité équivalente d'acide acrylamidoglycolique cristallisé avec une molécule d'eau.

On obtient ainsi 259g (1,5 mole) de MAGME cristallisé, incolore, présentant un point de fusion de 73°C (étalon F = 76°C).

Dosage de double liaison : 5,6 ± 0,04 méq/g soit 97 ± 1% de la théorie.

Chromatographie liquide à hautes performances :

```
. Présence de 96% de MAGME
        "       de  2% d'acide acryloylamino-2 méthoxy-2 acétique
        "       de 1,8% d'acryloylamino-2 hydroxy-2 acétate de
                   méthyle
        "       de 0,2% de bisacrylamidoacétate de méthyle.
```

Ce produit n'a pu être purifié par recristallisation dans du trichloro-1,1,1 éthane. Ainsi, le procédé de l'invention appliqué à l'acide acrylamidoglycolique cristallisé avec une molécule d'eau permet d'obtenir un MAGME dont la pureté n'est que de 96% avec un rendement de 75% de la théorie.

EXEMPLE DE COMPARAISON

On reproduit soigneusement le procédé décrit dans l'exemple 1 du brevet européen n° 0.020.000. On obtient ainsi 264g de MAGME brut présentant un point de fusion peu net d'environ 66 ± 2°C.

L'analyse par chromatographie liquide à hautes performances de ce produit révèle la présence de :

. 80% de MAGME

4% d'acryloylamino-2 hydroxy-2 acétate de méthyle

1,7% d'eau (déterminée par Karl Fisher)

1,5% d'acrylamide

0,2% d'AAG

0,5% d'acide acryloylamino-2 méthoxy-2 acétique

0,1% d'acide glyoxylique

12 % d'impuretés non identifiées.

Ce produit n'a pu être purifié, ni par distillation, ni par recristallisation. Le rendement s'établit à 83% de la théorie calculée par rapport à l'AAG mis en oeuvre pour un produit à 80% de pureté.

**Revendications**
**Revendications pour les Etats contractants suivants : AT, BE, CH, DE, FR, GB, GR, IT, LI, LU, NL, SE**

1. Acryloylamino-2 méthoxy-2 acétate de méthyle cristallisé pur sous forme de cristaux incolores présentant un point de fusion de 76°C.

2. Procédé d'obtention de l'acryloylamino-2 méthoxy-2 acétate de méthyle cristallisé pur caractérisé par le fait que l'on fait réagir, en milieu anhydre, de l'acide acrylamidoglycolique cristallisé pur anhydre avec du méthanol en présence de chlorure d'hydrogène, que l'on neutralise ensuite le milieu réactionnel avec un hydrogènocarbonate de métal alcalin puis que l'on filtre les sels minéraux et qu'enfin l'on isole l'acryloylamino-2 méthoxy-2 acétate de méthyle cristallisé pur par reprise du milieu réactionnel concentré sous vide avec du trichloro-1,1,1-éthane.

3. Procédé selon à revendication 2, caractérisé par le fait que l'on fait réagir, en milieu anhydre, à une température comprise entre 30 et 35°C, une mole d'acide acrylamidoglycolique cristallisé pur anhydre avec plus de 10 moles de méthanol en présence d'une mole de chlorure d'hydrogène anhydre, que l'on neutralise ensuite le milieu réactionnel avec de l'hydrogènocarbonate de sodium puis que l'on filtre les sels minéraux, que l'on élimine le méthanol en excès et l'eau formée d'abord par distillation sous vide à une température inférieure à 35°C puis, par distillation azéotropique sous vide à une température inférieure à 35°C avec du trichloro-1,1,1, éthane et que l'on isole enfin l'acryloylamino-2 méthoxy-2 acétate de méthyle par cristallisation dans du trichloro-1,1,1 éthane.

**Revendications pour l'Etat contractant suivant : ES**

1. Procédé d'obtention de l'acryloylamino-2 méthoxy-2 acétate de méthyle cristallisé pur caractérisé par le fait que l'on fait réagir, en milieu anhydre, de l'acide acrylamidoglycolique cristallisé pur anhydre avec du méthanol en présence de chlorure d'hydrogène, que l'on neutralise ensuite le milieu réactionnel avec un hydrogènocarbonate de métal alcalin puis que l'on filtre les sels minéraux et qu'enfin l'on isole l'acryloylamino-2 méthoxy-2 acétate de méthyle cristallisé pur par reprise du milieu réactionnel concentré sous vide avec du trichloro-1, 1, 1-éthane.

2. Procédé selon la revendication 1, caractérisé par le fait que l'on fait réagir, en milieu anhydre, à une température comprise entre 30 et 35°C, une mole d'acide acrylamidoglycolique cristallisé pur anhydre avec plus de 10 moles de méthanol en présence d'une mole de chlorure d'hydrogène anhydre, que l'on neutralise ensuite le milieu réactionnel avec de l'hydrogènocarbonate de sodium puis que l'on filtre les sels minéraux, que l'on élimine le méthanol en excès et l'eau formée d'abord par distillation sous vide à une température inférieure à 35 °C puis, par distillation azéotropique sous vide à une température inférieure à 35°C avec du trichloro-1,1,1-éthane et que l'on isole enfin l'acryloylamino-2 méthoxy-2 acétate de méthyle par cristallisation dans du trichloro-1, 1, 1-éthane.

**Claims**
**Claims for the following Contracting States : AT, BE, CH, DE, FR, GB, GR, IT, LI, LU, NL, SE**

1. Crystalline methyl 2-acrylylamino-2-methoxy acetate in the form of colourless crystals having a melting point of 76°C

2. A process for the preparation of pure crystalline methyl 2-acrylylamino-2-methoxy acetate by reacting pure crystalline anhydrous acrylamidoglycolic acid in an anhydrous medium with methanol in the presence of hydrogen chloride, whereafter the reaction medium is neutralized with an alkaline metal bicarbonate and then the mineral salts are filtered off and finally the pure crystalline methyl 2-acrylylamino-2-methoxy acetate is isolated by taking up the concentrated reaction medium with 1,1,1-trichloroethane in vacuo.

3. A process according to claim 2, characterized in that one mole of pure crystalline anhydrous acrylamidoglycolic acid is reacted in an anhydrous medium at a temperature of between 30 and 35°C with more than 10 moles of methanol in the presence of one mole of anhydrous hydrogen chloride, whereafter the reaction medium is neutralized using sodium bicarbonate and the mineral salts are filtered off, the excess methanol and water formed are eliminated by initial vacuum distillation at a temperature below 35°C, then by azeotropic distillation in vacuo with 1,1,1-trichloroethane at a temperature below 35°C, and finally the methyl 2-acrylylamino-2-methoxy acetate is isolated by crystallization from 1,1,1-trichloroethane.

**Claims for the following Contracting State : ES**

1. A process for the preparation of pure crystalline methyl 2-acrylylamino-2-methoxy acetate, characterized in that pure crystalline anhydrous acrylamidoglycolic acid is reacted in an anhydrous medium with methanol in the presence of hydrogen chloride, whereafter the reaction medium is neutralized with an alkaline metal bicarbonate and then the mineral salts are filtered off and finally the pure crystalline methyl 2-acrylylamino-2-methoxy acetate is isolated by taking up the concentrated reaction medium with 1,1,1-trichloroethane in vacuo.

2. A process according to claim 1, characterized in that one mole of pure crystalline anhydrous acrylamidoglycolic acid is reacted in an anhydrous medium at a temperature of between 30 and 35°C with more than 10 moles of methanol in the presence of one mole of anhydrous hydrogen chloride, whereafter the reaction medium is neutralized using sodium bicarbonate and the mineral salts are filtered off, the excess methanol and water formed are eliminated by initial distillation in vacuo at a temperature below 35°C and then by azeotropic distillation in vacuo with 1,1,1-trichloroethane at a temperature below 35°C, and finally the methyl 2-acrylylamino-2-methoxy acetate is isolated by crystallization from 1,1,1-trichloroethane.

**Patentansprüche**
**Patentansprüche für folgende Vertragsstaaten : AT, BE, CH, DE, FR, GB, GR, IT, LI, LU, NL, SE**

1. Reines, kristallines 2-Acryloylamino-2-methoxy-methylacetat in Form von farblosen Kristallen mit einem Schmelzpunkt von 76°C.

2. Verfahren zur Herstellung von reinem, kristallinem 2-Acryloylamino-2-methoxy-methylacetat, dadurch gekennzeichnet, daß wasserfreie reine, kristalline Acrylamidoglykolsäure in wasserfreiem Medium mit Methanol in Gegenwart von Chlorwasserstoff reagieren gelassen wird, danach das Reaktionsmedium mit einem Alkalimetallhydrogencarbonat neutralisiert wird, dann die Mineralsalze abfiltriert werden, und schließlich durch Aufnahme des konzentrierten Reaktionsmediums in 1,1,1-Trichlorethan unter Vakuum das reine, kristalline 2-Acryloylamino-2-methoxy-methylacetat isoliert wird.

3. Verfahren nach Anspruch 2, dadurch gekennzeichnet, daß ein Mol wasserfreie reine, kristalline Acrylamidoglycolsäure mit mehr als 10 Mol Methanol in Gegenwart von einem Mol wasserfreiem Chlorwasserstoff in wasserfreiem Medium bei einer Temperatur von 30 bis 35°C reagieren gelassen wird, danach das Reaktionsmedium mit Natriumhydrogencarbonat neutralisiert wird, dann die Mineralsalze abfiltriert werden, daß das überschüssige Methanol und das gebildete Wasser zuerst durch Destillation unter Vakuum bei einer Temperatur von weniger als 35°C und dann durch azeotrope Destillation unter Vakuum bei einer Temperatur von weniger als 35°C mit 1,1,1-Trichlorethan entfernt werden, und daß schließlich 2-Acryloylamino-2-methoxy-methylacetat durch Kristallisation in 1,1,1-Trichlorethan isoliert wird.

**Patentansprüche für folgenden Vertragsstaat : ES**

1. Verfahren zur Herstellung von reinem, kristallinem 2-Acryloylamino-2-methoxy-methylacetat, dadurch gekennzeichnet, daß wasserfreie reine, kristalline Acrylamidoglykolsäure in wasserfreiem Medium mit Methanol in Gegenwart von Chlorwasserstoff reagieren gelassen wird, danach das Reaktionsmedium mit einem Alkalimetallhydrogencarbonat neutralisiert wird, dann die Mineralsalze abfiltriert werden, und schließlich durch Aufnahme des konzentrierten Reaktionsmediums in 1,1,1-Trichlorethan unter Vakuum das reine, kristalline 2-Acryloylamino-2-methoxy-methylacetat isoliert wird.

2. Verfahren nach Anspruch 2, dadurch gekennzeichnet, daß ein Mol wasserfreie reine, kristalline Acrylamidoglycolsäure mit mehr als 10 Mol Methanol in Gegenwart von einem Mol wasserfreiem Chlorwasserstoff in wasserfreiem Medium bei einer Temperatur von 30 bis 35°C reagieren gelassen wird, danach das Reaktionsmedium mit Natriumhydrogencarbonat neutralisiert wird, dann die Mineralsalze abfiltriert werden, daß das überschüssige Methanol und das gebildete Wasser zuerst durch Destillation unter Vakuum bei einer Temperatur von weniger als 35°C und dann durch azeotrope Destillation unter Vakuum bei einer Temperatur von weniger als 35°C mit 1,1,1-Trichlorethan entfernt werden, und daß schließlich 2-Acryloylamino-2-methoxy-methylacetat durch Kristallisation in 1,1,1-Trichlorethan isoliert wird.